# EUROPEAN PATENT APPLICATION

(11) **EP 4 702 945 A2**
(43) Date of publication of application: **04.03.2026**
(21) Application number: 25197210.5
(22) Date of filing: 21.08.2025
(51) Int. Cl.: A61B 34/10, A61B 5/00, A61B 17/17, A61F 2/38, A61B 34/20, A61B 90/00

(54) **PATELLA TRACKER AND PLANNING**

(30) Priority: 28.08.2024 US 202463688017 P
(71) Applicant: Howmedica Osteonics Corp., Mahwah, NJ 07430 (US)
(72) Inventor: HAMPP, Emily, FAR HILLS, 07931 (US); ALI, Azhar, WEST ORANGE, 07052 (US); LUSTIG, Sebastien, 69370 SAINT DIDIER AU MONT D'OR (FR)
(74) Representative: Regimbeau

(57) **Abstract**

Disclosed herein is a method for patella planning. The method can include tracking a location of a point adjacent an unresected patella in flexion and extension of a knee, generating a native kinematic profile from the location of the point, and displaying and comparing the native kinematic profile with a target kinematic profile. The native kinematic profile can represent a kinematic profile of the unresected patella. The method can include using the native kinematic profile prior to bone resections if the native kinematic profile is within predetermined thresholds of the target kinematic profile, performing pre-resection steps prior to bone resections if the native kinematic profile is outside predetermined thresholds of the target kinematic profile.

## Description

### FIELD OF INVENTION

The present invention relates to a surgical system and a method for tracking a patella and planning a total knee arthroplasty, and more particularly to a surgical system and a method for tracking a patella to assess patellofemoral joint kinematics and planning a total knee arthroplasty.

### BACKGROUND OF THE INVENTION

Intra-operative assessment of the patellofemoral joint (PFJ) in total knee arthroplasty (TKA) is particularly challenging due to the complex biomechanics involved. Maintaining natural or desired biomechanics of the PFJ is critical for proper rehabilitation and function of the knee joint. Precise implant sizing and positioning are essential to ensure that postoperative patellar kinematics closely resemble the patient's preoperative state or a desired state. However, improper sizing or positioning of patellar implants can result in patellar maltracking and other complications.

Current methods of selecting patellar implants based on a limited number of knee flexion-extension positions do not necessarily ensure proper patellar kinematics throughout the entire range of motion. The patellar range of motion involves movement with six degrees of freedom during the flexion-extension cycle. Consequently, selecting patellar implants based on limited positional data may be inadequate. Anatomically shaped patellar implants, while often providing better rehabilitation outcomes, present additional challenges in sizing due to their complex geometric configuration and range of motion.

To improve surgical outcomes, additional tools and methods are required to quantify the position of the patella relative to the femur throughout the range of motion. This quantification will improve surgical decisions regarding the positioning and selection of patellar and femoral implants.

Therefore, there exists a need for a surgical system and method to assess a knee joint intra-operatively for precise knee implant implant positioning and selection.

### BRIEF SUMMARY OF THE INVENTION

According to the invention there is provided a method for patella planning as defined in the appended claims.

Disclosed herein are surgical systems for bone tracking and methods for implant selection. In one embodiment, a surgical method utilizing a surgery system configured to enhance planning, accuracy, and outcomes in knee surgeries, particularly total knee arthroplasty (TKA) is disclosed. The system facilitates the assessment of native, trialing, and final patellar kinematics by employing imaging and tracking technologies to provide real-time data on the position and movement of the patella. It dynamically tracks the patella through its range of motion, capturing multiple data points to accurately determine the patient's native patellofemoral kinematic profile or pattern. This detailed kinematic map can be compared to a target profile to serve as a benchmark for optimal joint function. The system offers a visual display of the planned kinematic profile based on the specific implant plan and suggests modifications if deviations occur, including adjustments to implant position, size, and component type. The ability to assess and adjust the kinematic profile intra-operatively ensures optimal alignment and function of the knee joint post-surgery, reducing the risk of complications and improving overall outcomes of TKA procedures.

In an aspect of the present disclosure, a method for patella planning is provided. A method according to this aspect may include the steps of tracking a location of a point adjacent an unresected patella in flexion and extension of a knee, generating a native kinematic profile from the location of the point, and displaying and comparing the native kinematic profile with a target kinematic profile. The native kinematic profile may represent a kinematic profile of the unresected patella.

Continuing in accordance with this aspect, the method may further include a step of using the native kinematic profile prior to bone resections if the native kinematic profile is within predetermined thresholds of the target kinematic profile.

Continuing in accordance with this aspect, the method may include a step of performing pre-resection steps prior to bone resections if the native kinematic profile is outside predetermined thresholds of the target kinematic profile. The pre-resection steps may include any of resurfacing the patella, adjusting a patella resection depth, adjusting a patellar implant thickness, adjusting a patellar implant location, and adjusting femur and tibia positions.

Continuing in accordance with this aspect, the native kinematic profile and the target kinematic profile may represent patella anterior-posterior translation.

Continuing in accordance with this aspect, the native kinematic profile and the target kinematic profile may represent patella medial-lateral translation.

Continuing in accordance with this aspect, the native kinematic profile and the target kinematic profile may represent patella superior-inferior translation.

Continuing in accordance with this aspect, the native kinematic profile and the target kinematic profile may represent patella medial-lateral tilt.

Continuing in accordance with this aspect, the native kinematic profile and the target kinematic profile may represent patella superior-inferior tilt.

Continuing in accordance with this aspect, the native kinematic profile and the target kinematic profile may represent patella varus-valgus rotation.

Continuing in accordance with this aspect, the target kinematic profile may represent a kinematic profile of a healthy patella.

Continuing in accordance with this aspect, the step of tracking the location of the point adjacent the unresected patella may include tracking a plurality of points adjacent the unresected patella in flexion and extension of the knee.

Continuing in accordance with this aspect, the step of displaying and comparing the native kinematic profile with the target kinematic profile may include displaying and comparing the native kinematic profile with the target kinematic profile on a display screen.

Continuing in accordance with this aspect, the target kinematic profile may be defined by predetermined thresholds.

Continuing in accordance with this aspect, the step of generating the native kinematic profile may include displaying multiple points representing locations of the tracked points on a display screen.

Continuing in accordance with this aspect, the step of generating the native kinematic profile may include displaying a line connecting locations of the tracked points on a display screen.

In accordance with another aspect of the present disclosure, a method for patella planning is provided. A method according to this aspect may include the steps of resecting a femur, tracking a location of a point adjacent a resurfaced patella in flexion and extension of a knee, generating a native kinematic profile from the location of the point, and displaying and comparing the native kinematic profile with a target kinematic profile. The native kinematic profile may represent a kinematic profile of the resurfaced patella.

Continuing in accordance with this aspect, the method may include a step of performing post-resection steps prior if the native kinematic profile is outside predetermined thresholds of the target kinematic profile. The post-resection steps may include any of adjusting a patella resection depth, adjusting a patellar implant thickness, adjusting a patellar implant location, and adjusting femur and tibia positions.

Continuing in accordance with this aspect, the native kinematic profile and the target kinematic profile may represent patella anterior-posterior translation.

Continuing in accordance with this aspect, the native kinematic profile and the target kinematic profile may represent patella medial-lateral translation.

Continuing in accordance with this aspect, the native kinematic profile and the target kinematic profile may represent patella superior-inferior translation.

Continuing in accordance with this aspect, the native kinematic profile and the target kinematic profile may represent patella medial-lateral tilt.

Continuing in accordance with this aspect, the native kinematic profile and the target kinematic profile may represent patella superior-inferior tilt.

Continuing in accordance with this aspect, the native kinematic profile and the target kinematic profile may represent patella varus-valgus rotation.

Continuing in accordance with this aspect, the target kinematic profile may represent a kinematic profile of a healthy patella.

Continuing in accordance with this aspect, the step of tracking the location of the point adjacent the resurfaced patella may include tracking a plurality of points adjacent the resurfaced patella in flexion and extension of the knee.

Continuing in accordance with this aspect, the step of displaying and comparing the native kinematic profile with the target kinematic profile may include displaying and comparing the native kinematic profile with the target kinematic profile on a display screen.

Continuing in accordance with this aspect, he target kinematic profile may be defined by predetermined thresholds.

Continuing in accordance with this aspect, the step of generating the native kinematic profile may include displaying multiple points representing locations of the tracked points on a display screen.

Continuing in accordance with this aspect, the step of generating the native kinematic profile may include displaying a line connecting locations of the tracked points on a display screen.

### BRIEF DESCRIPTION OF THE DRAWINGS

A more complete appreciation of the subject matter of the present invention and the various advantages thereof can be realized by reference to the following detailed description, in which reference is made to the following accompanying drawings:
FIG. 1 is a flowchart of a surgical method using a surgical system in accordance with an embodiment of the present disclosure;
FIG. 2 is a schematic side view showing tracker positions of a patella according to an embodiment of the present disclosure.
FIG. 3 is a graphical representation of a femur showing paths defined by the trackers of FIG. 2 on a display;
FIG. 4 is a graphical representation showing safe zones for patella kinematics according to an embodiment of the present disclosure;
FIG. 5 is a flowchart showing pre-resection considerations of FIG. 1;
FIG. 6 is a schematic view of an anterior-posterior (AP) translation of a patella;
FIG. 7 is a schematic view of a medial-lateral (ML) translation of a patella;
FIG. 8 is a schematic view of a superior-inferior (SI) translation of a patella;
FIG. 9 is a schematic view of a medial-lateral (ML) tilt of a patella;
FIG. 10 is a schematic view of a superior-inferior (SI) tilt of a patella;
FIG. 11 is a schematic view of varus-valgus (VV) rotation of a patella;
FIG. 12 is a graph showing tracked patella AP translation with respect to knee flexion according to an embodiment of the present disclosure;
FIG. 13 is a flowchart showing surgical steps in response to the tracked patella AP translation according to an embodiment of the present disclosure;
FIG. 14 is a graph showing tracked patella ML tilt with respect to knee flexion according to an embodiment of the present disclosure;
FIG. 15 is a flowchart showing surgical steps in response to the tracked patella ML tilt according to an embodiment of the present disclosure;
FIG. 16 is a graph showing tracked patella SI translation with respect to knee flexion according to an embodiment of the present disclosure;
FIG. 17 is a flowchart showing surgical steps in response to the tracked patella SI translation according to an embodiment of the present disclosure;
FIG. 18 is a graph showing tracked patella ML translation with respect to knee flexion according to an embodiment of the present disclosure;
FIG. 19 is a flowchart showing surgical steps in response to the tracked patella ML translation according to an embodiment of the present disclosure;
FIG. 20 is a graph showing tracked patella SI tilt with respect to knee flexion according to an embodiment of the present disclosure;
FIG. 21 is a flowchart showing surgical steps in response to the tracked patella SI tilt according to an embodiment of the present disclosure, and
FIG. 22 is a flowchart showing surgical steps in response to a tracked patella VV rotation according to an embodiment of the present disclosure.

### DETAILED DESCRIPTION

Reference will now be made in detail to the various embodiments of the present disclosure illustrated in the accompanying drawings. Wherever possible, the same or like reference numbers will be used throughout the drawings to refer to the same or like features within a different series of numbers (e.g., 100-series, 200-series, etc.). It should be noted that the drawings are in simplified form and are not drawn to precise scale. Additionally, the term "a," as used in the specification, means "at least one." The terminology includes the words above specifically mentioned, derivatives thereof, and words of similar import. Although at least two variations are described herein, other variations may include aspects described herein combined in any suitable manner having combinations of all or some of the aspects described.

As used herein, the terms "tracker" and "marker" will be used interchangeably and as such, unless otherwise stated, the explicit use of either term is inclusive of the other term. Similarly, the terms "patellofemoral kinematics" and "patella kinematics" will be used interchangeably and as such, unless otherwise stated, the explicit use of either term is inclusive of the other term.

In describing preferred embodiments of the disclosure, reference will be made to directional nomenclature used in describing the human body. It is noted that this nomenclature is used only for convenience and that it is not intended to be limiting with respect to the scope of the present disclosure. As used herein, when referring to bones or other parts of the body, the term "anterior" means toward the front part of the body or the face, and the term "posterior" means toward the back of the body. The term "medial" means toward the midline of the body, and the term "lateral" means away from the midline of the body. The term "superior" means closer to the head, and the term "inferior" means more distant from the head.

Disclosed herein is a surgical method, which utilizes a surgical system designed to enhance planning, accuracy, and outcomes in knee surgeries, such as total knee arthroplasty (TKA). This surgical system facilitates the assessment of native, trialing, and final patellar kinematics, allowing surgeons to make informed decisions throughout the procedure. The system employs imaging and tracking technologies to provide real-time data on the position and movement of the patella. For example, the system can dynamically track the patella by holding a probe against it while moving the knee through its range of motion. This process involves capturing multiple data points to accurately determine the patient's native patellofemoral kinematic profile.

The system's capability to capture and analyze these data points enables it to construct a detailed map of the patella's motion relative to the femur. This native kinematic profile can then be compared to a target kinematic profile, which serves as a benchmark for optimal joint function. Additionally, the system provides a visual display of a planned patellofemoral kinematic profile based on the specific implant plan. If this planned profile deviates from the desired safe zone relative to the target profile, the system can suggest modifications to the implant plan. Adjustments may include changes to the planned implant position, size, and component type. For instance, the system may recommend altering the thickness of the patella or adjusting the location of the patellar apex to bring the planned kinematic profile back into the safe zone.

The ability to dynamically assess and adjust the kinematic profile intra-operatively ensures that the surgical plan can be optimized intra-operatively to better alignment and function of the knee joint post-surgery. By providing this level of detailed analysis and real-time feedback, the surgical system reduces the risk of complications such as patellar maltracking and to improve the overall outcomes of TKA procedures.

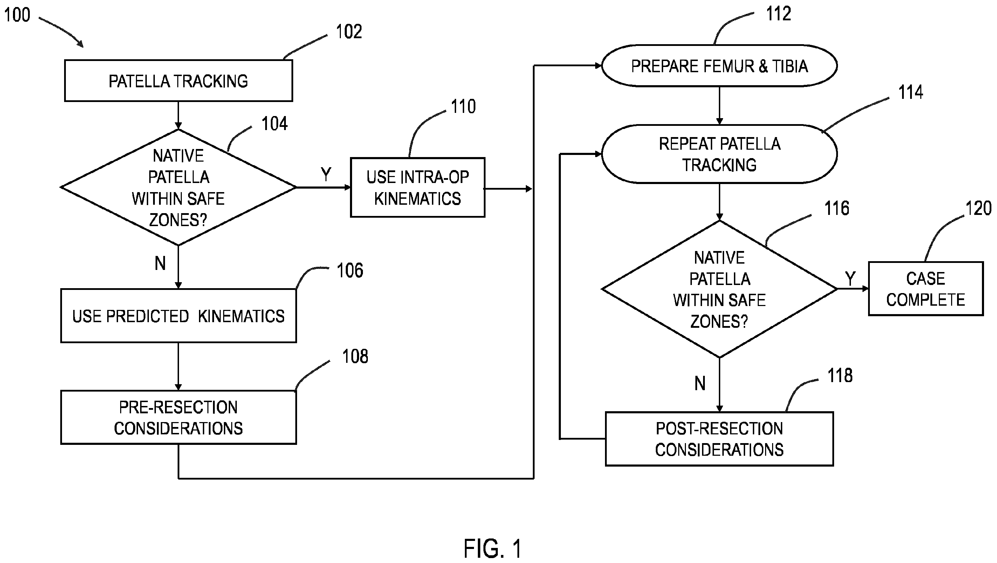

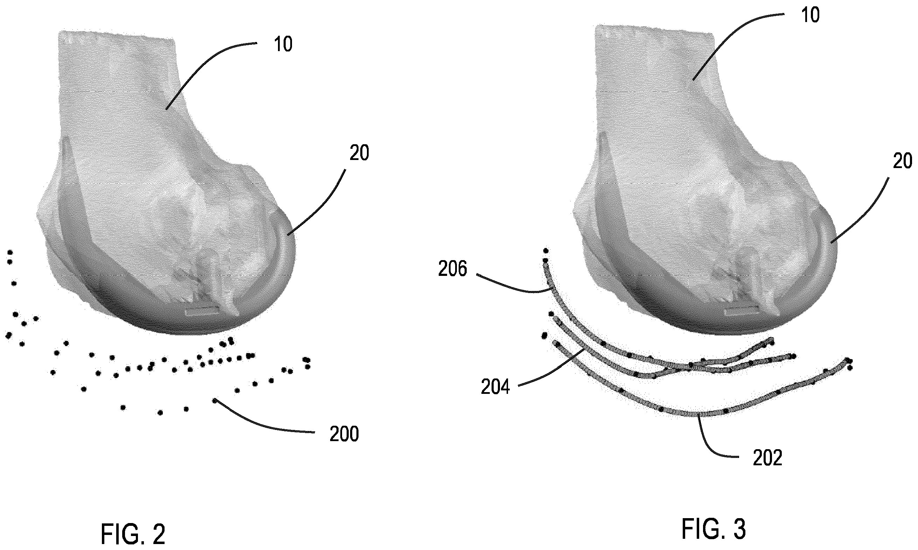

Referring now to FIG. 1, there is a shown a surgical method 100 using a surgical system in accordance with an embodiment of the present disclosure. Surgical method 100 allows improves the planning, accuracy, and outcomes in knee surgeries, particularly total knee arthroplasty (TKA), using the surgical system. Surgical method 100 details the process of patella tracking and decision-making to ensure optimal patellar kinematics. The surgical system can include trackers and a tracking system with a position capturing device and display. It can track bone positions 200 using markers on trackers that contact bones like a patella and/or a femur 20 (FIG. 2). The system can dynamically record and display precise bone movements during surgeries, including bone paths (202, 204, 206) derived from bone positions 200 as shown in FIG. 3. It should be noted that while paths 202, 204, 206 are depicted as lines in FIG. 3, generating lines connecting the tracker positions is not necessary for the purposes of patellofemoral kinematic analysis as disclosed herein. In other words, the tracker locations alone are sufficient for the surgical system to perform the various functions described including generation of the kinematic profiles.

A step 102 for patella tracking of surgical method 100 can include several actions. Initially, an operator or surgeon completes bone registration and assessments using the surgical system with the anterior cruciate ligament (ACL) intact. Then, the implant plan can be adjusted based on Functional Knee Positioning Principles, ensuring optimal alignment, muscle balance, and joint stability. This can involve aligning the knee with the hip and ankle, balancing surrounding muscles, promoting controlled movements, managing load to prevent overloading, maintaining flexibility, incorporating functional exercises, and using biomechanical assessments to correct any movement deviations. The surgeon can then prepare the patella by placing three checkpoints or marking three points, for example, on the medial edge, lateral edge, and inferior pole of the dorsal face. This is followed by capturing patellar center points at varying degrees of flexion relative to the femur using a probe. The system is then used to digitize three points (medial, lateral, and inferior edge) relative to the tibia and the dorsal surface of the patella, ensuring the knee remains immobile. The surgeon then places the probe on the patellar medial edge and takes a screenshot during a dynamic flexion cycle from 120 to 10 degrees, repeating for the lateral edge and inferior pole. If the ACL and meniscus are removed, the surgeon can optionally repeat these steps. Thus, a user can hold a probe against the patella while moving the knee through its range of motion to collect data points and determine the patient's native patellofemoral kinematics.

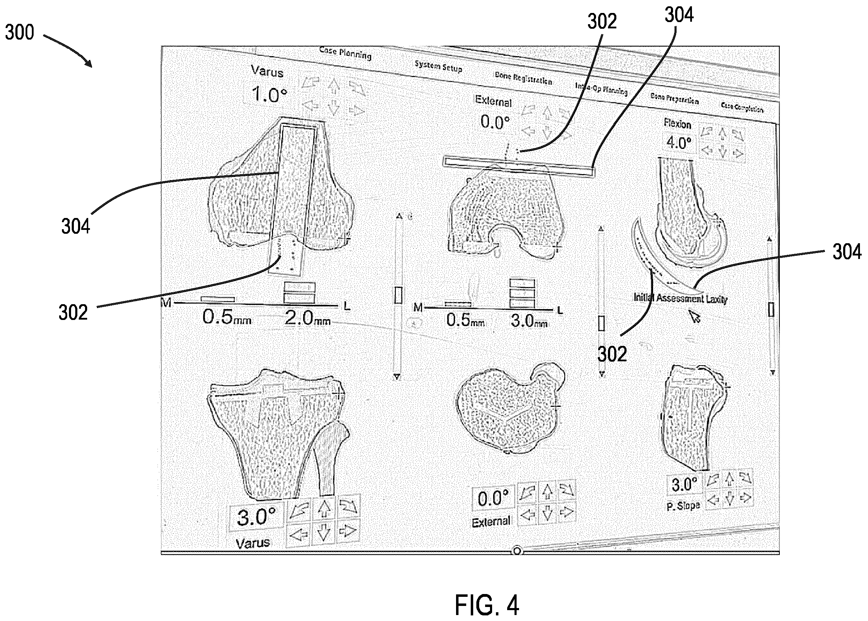

In a step 104 of surgical method 100, the measured patellofemoral kinematics is compared with predetermined thresholds, as best shown in FIG. 4. A decision point at step 104 checks whether the native patella kinematic profile is within predefined safe zones 304. These safe zones represent the allowable deviation between the planned (or final) and target kinematic profiles 302 as displayed on display screen 300 of FIG. 4. The assessment can include evaluating whether the native patella anatomy is healthy using methods such as B-score, C-score, CT, or visual assessment, and whether the native patella tracking is within the safe zones using a predicted AI or morphology-driven model. The patient's native patellofemoral kinematic profile can be compared to a target profile. Additionally, a planned patellofemoral kinematic profile can be displayed based on the specific implant plan as more fully disclosed below. If the planned kinematic profile falls outside of the desired safe zone relative to the target profile, adjustments can be made by modifying the planned implant position, size, and component type. For example, the patella thickness and the location of the patellar apex may be adjusted to bring the planned kinematic profile back into the safe zone. There can be typically two desired target patellar kinematic profiles based on the current disease state of the patellofemoral anatomy: (1) a patient's native kinematic profile (pre-resection) for normal patellofemoral anatomy, which can be computed intraoperatively using dynamic assessment, and (2) a patient's neutral kinematic profile for diseased patellofemoral anatomy, which can be automatically determined from trochlear bony anatomy using CT.

Safe zones 304 for the target kinematic profiles can be displayed in the frontal, sagittal, and axial planes, representing the allowable deviation between the planned (or final) and target kinematic profiles 302 as shown in FIG. 4. These safe zones can be established by calculating the average deviation from the native or neutral paths from 0 to 90 degrees of flexion, in 30-degree increments (0 to 30, 30 to 60, and 60 to 90 degrees), with a weighted emphasis on mid-flexion (30 to 60 degrees). Additionally, safe zones can be customized based on surgical preferences, establishing tolerances between the trochlear medial and lateral apexes.

If the patient's patellofemoral anatomy and kinematics are within desired limits, the surgeon can restore the patient's native kinematics profile in a step 110. This ensures that the planned surgical outcomes closely align with the patient's natural knee function. Thus, if the native patella kinematics is within the safe zones, the system utilizes intra-operative kinematics to guide the surgical procedure. This step involves the use of real-time data to make precise adjustments during the surgery. The intra-operative kinematics provide continuous feedback on the patella's position and movement, allowing for dynamic adjustments as needed.

If the native patella kinematic profile is not within the safe zones, the system uses predicted kinematics (step 106). This can include utilizing pre-operative imaging and planning data to predict the kinematic behavior of the patella. This process can begin with comprehensive pre-operative imaging, such as CT or MRI, to create a 3D model of the patient's knee anatomy. The imaging data can be analyzed to develop a kinematic model that simulates the patella's motion relative to the femur and tibia, considering the unique anatomical features and current condition of the joint. Software algorithms can be used to process this data to predict the patellar behavior throughout the knee's range of motion, identifying potential deviations from the optimal kinematic path.

The system can use the predicted kinematic profiles derived from either an AI model or a morphology-driven model. The AI model can leverage machine learning algorithms trained on a large dataset of healthy knee kinematics to predict the optimal patellar movement. The morphology-driven model may use a closest-point calculation of the patella crest to the femoral trochlear groove to determine the best kinematic path. These adjustments are validated through simulation to ensure they will bring the patellar movement within the safe zones, aiming to achieve optimal kinematic outcomes and reduce the risk of postoperative complications.

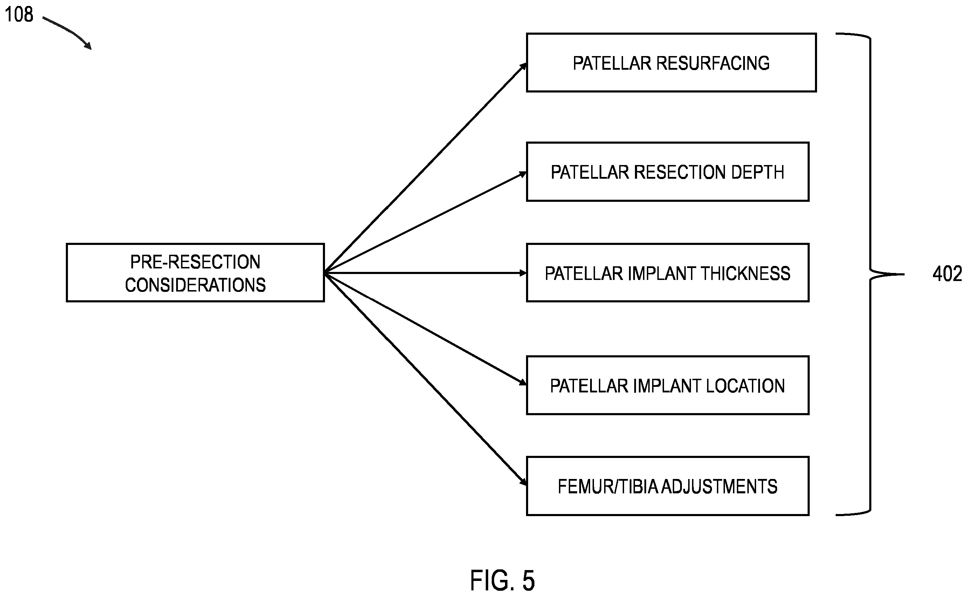

Before proceeding with the bone resections, several adjustments 400 can be made to ensure proper patellofemoral kinematics in step 108 if predicted kinematics are utilized. These steps, as shown in FIG. 5, can include patellar resurfacing, determining the patellar resection depth, adjusting the patellar implant thickness, deciding the patellar implant location, making necessary femoral and tibial adjustments, etc. Patellar resurfacing can include reshaping or resurfacing the patella to create a smooth surface that interacts optimally with the femoral component. This process may involve removing damaged or diseased cartilage and bone to prepare the patella for implant placement.

Adjustments to the patella's position in various directions are necessary for proper tracking. These adjustments can include anterior/posterior (A/P) translations, which control the patella's forward or backward position, ensuring it does not sit too far in either direction, potentially affecting overall knee mechanics. Superior/inferior (S/I) adjustments correct the patella's height along the vertical axis, aligning it correctly with the femoral trochlea to prevent issues like patellar baja (low-riding patella) or patellar alta (high-riding patella). Medial/lateral (M/L) adjustments fine-tune the patella's side-to-side position, ensuring it tracks within the central groove of the femoral component, thus preventing lateral or medial patellar subluxation or dislocation.

Rotational adjustments, such as varus/valgus (V/V) rotations, align the patella implant to match the anatomical shape of the knee, ensuring smooth movement over the femoral trochlea without excessive tilting. Additionally, choosing the appropriate implant type significantly influences the outcomes. Dome implants, with their simplified geometry, offer consistent tracking but may not conform to the native anatomy as closely as anatomic designs. Anatomic implants, designed to mimic the natural shape of the patella, provide improved kinematics and a more natural feel, requiring precise positioning to align with the patient's native anatomy.

Besides adjustments to the patella, modifications to the femur or tibia may be necessary. Adjusting the positioning of the femoral and tibial components ensures proper interaction with the patellar implant. This involves making changes to the anterior-posterior, medial-lateral, or proximal-distal positioning of these components. The choice of femoral and tibial implants also impacts overall knee kinematics, as different designs offer varying degrees of conformity and stability. Adjusting the alignment boundaries or the angles at which the femoral and tibial components are placed optimizes the knee's mechanical axis, ensuring proper patellar tracking and smooth movement. These comprehensive adjustments and modifications collectively aim to achieve optimal patellofemoral kinematics, improving the surgical outcomes and the patient's postoperative functionality.

In a next step 112, the femur and tibia are prepared for implant placement according to the optimized surgical plan. This preparation includes resecting the bones to create the appropriate surfaces for the implants, ensuring proper alignment and fit based on pre-planned measurements and positions. Precise bone cuts are made to remove damaged or diseased bone and to shape the remaining bone to match the contours of the planned implants. Cutting guides and alignment tools are used to ensure accuracy during the resection process.

After preparing the femur and tibia, patella tracking is repeated in a step 114. This step involves dynamically assessing the patella's motion again to collect updated kinematic data. The tracked patellofemoral kinematics are compared to the predefined safe zones in step 116. If the tracked kinematics fall within the safe zones, it indicates that the patellar movement is optimal, and the case is considered complete (step 120). This ensures that the patellofemoral kinematics are optimized for the patient, providing stability and proper function of the knee joint.

Alternatively, if the tracked patellofemoral kinematics are not within the safe zones, post-resection considerations are performed in a step 118. This step may involve making additional modifications to the implant positions, sizes, and types to achieve optimal patellar kinematics. Post-femoral and post-tibial resection considerations to achieve kinematic profiles within the safe zones can include patella resurfacing, adjustments to translations (e.g., anterior-posterior for resection depth, superior-inferior for patella height, medial-lateral), rotations (varus-valgus for anatomic implant type), implant type (e.g., dome, anatomic), re-cuts of the femur or tibia to adjust implant position, implant type, or alignment boundaries, and soft tissue releases, such as lateral retinacular release, etc.

Once these post-resection considerations have been performed, patella tracking is repeated to ensure that the adjustments have brought the kinematics within the safe zones. This iterative process of assessment and adjustment helps to achieve optimal patellar tracking and ensures the overall success of the knee arthroplasty procedure.

Surgical method 100 illustrates a systematic approach to dynamically assess and adjust the patellofemoral kinematics during TKA. Robotic tools can be used in conjunction with the surgical system for real-time data collection to enhance surgical precision and patient outcomes. By continuously monitoring and adjusting the patellar and femoral implant positions, the surgical system aims to reduce the risk of complications such as patellar maltracking and improve the overall success of the knee surgery. The surgical system helps identify and correct deviations from the safe zones, reducing the risk of complications such as patellar maltracking. This leads to better joint stability, overall success of the knee surgery, minimizes postoperative complications, and promotes faster recovery.

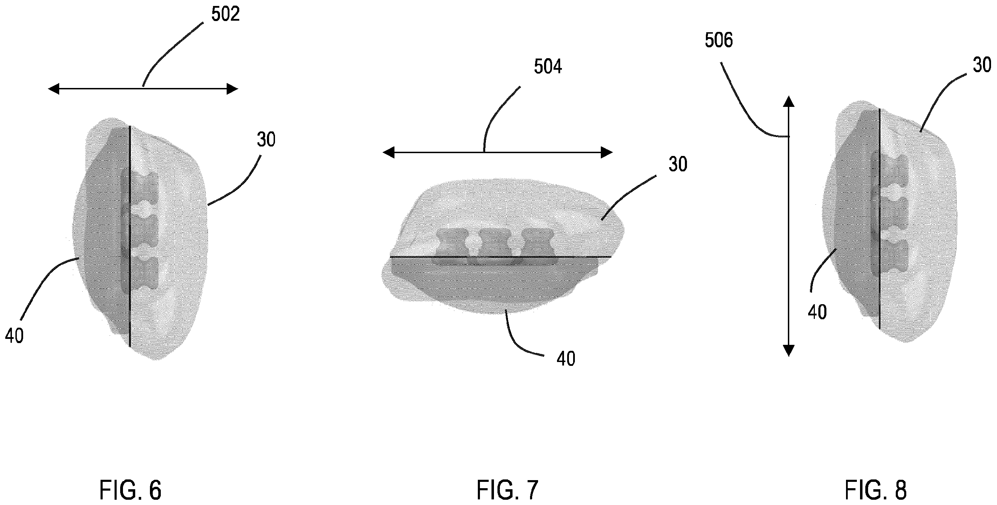

FIGS. 6 through 8 are schematic drawings illustrating the types of patellar translation during knee flexion and extension that can be tracked and utilized by the surgical system and methods disclosed herein. FIG. 6 shows a patella 30 with a patellar implant 40, with a line 502 depicting the anterior-posterior (AP) translation of the patella and the patellar implant. Notably, the system can also track the AP translation of the patella without the implant, providing flexibility in different surgical scenarios. FIG. 7 illustrates the medial-lateral (ML) translation of the patella, depicted by a line 504, which helps in understanding the side-to-side movement of the patella, crucial for ensuring proper alignment and avoiding maltracking. FIG. 8 depicts the superior-inferior (SI) translation of the patella, shown by line 506, highlighting the importance of monitoring this vertical movement for maintaining the correct height and positioning of the patellar implant.

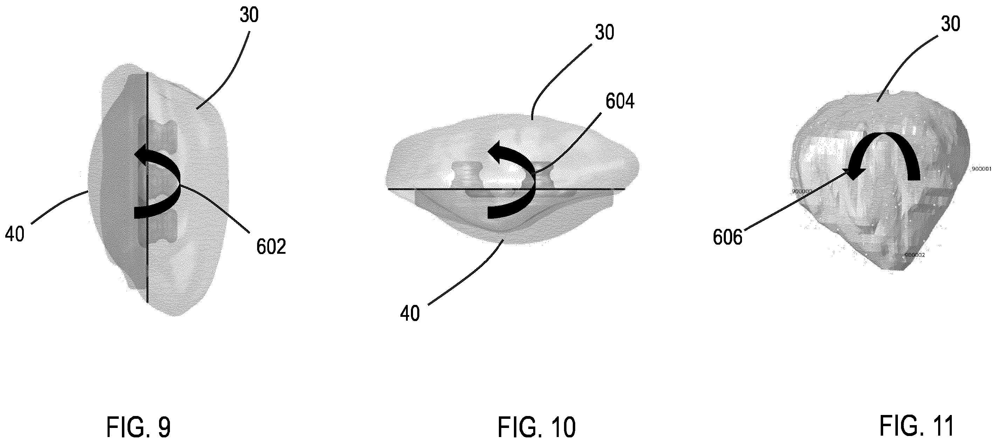

Referring now to FIGS. 9-11, schematic illustration of various rotational motions of the patella and the patellar implant during knee flexion and extension are shown. These rotational motions can be tracked and utilized within the disclosed system and methods. FIG. 9 depicts a medial-lateral tilt of the patella during flexion and extension, as indicated by a line 602. FIG. 10 shows the superior-inferior tilt of the patella, denoted by a line 604, highlighting the rotational motion essential for accurate implant positioning. FIG. 11 illustrates the varus-valgus tilt of the patella, depicted by line 606, emphasizing the importance of monitoring this tilt to ensure proper alignment and function of the knee joint. These figures collectively demonstrate how the surgical system tracks various translation motions of the patella, both with and without the patellar implant. This comprehensive tracking capability enhances the precision of the surgical procedure, ensuring optimal implant positioning and improving overall patient outcomes.

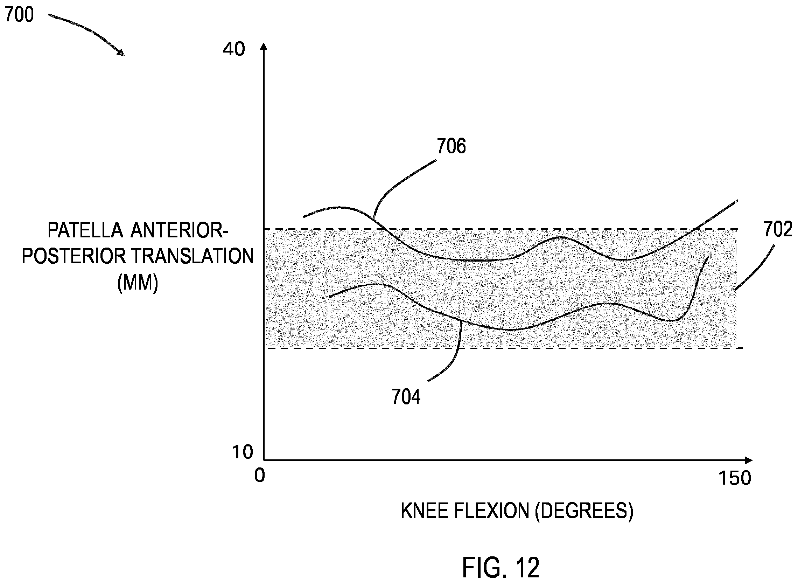

Graph 700 shown in FIG. 12, shows an anterior-posterior translation of the patella with respect to knee flexion and extension according to another embodiment of the present disclosure. Graph 700 tracks the movement of the patella and/or patellar implant as the knee flexes from 0 degrees to 150 degrees, providing kinematic data (AP translation) for the surgical system. The surgical system of the present disclosure monitors the patella's trackers and generates distinct paths, 704 and 706, as depicted in FIG. 12. Path 704 represents the anterior-posterior translation of the patella that remains within a designated safe zone 702 throughout the entire range of knee flexion and extension. This indicates that the patella's movement along path 704 is within acceptable limits, ensuring proper patellar kinematics and reducing the risk of complications such as patellar maltracking. Conversely, path 706 shows the anterior-posterior translation of the patella that deviates from the safe zone 702 in certain areas. These deviations suggest that the patellar movement along path 706 is suboptimal, requiring additional surgical intervention to align the patella's motion within the ideal kinematic parameters. This might involve adjusting the implant position, size, or type, or making further modifications to the surgical plan to achieve the desired patellar kinematics.

The safe zone can be defined, for example, as the range between 15 mm and 30 mm of anterior-posterior (AP) translation. If the tracked AP translation falls outside this range, it may indicate incorrect patellar motion that requires adjustment.

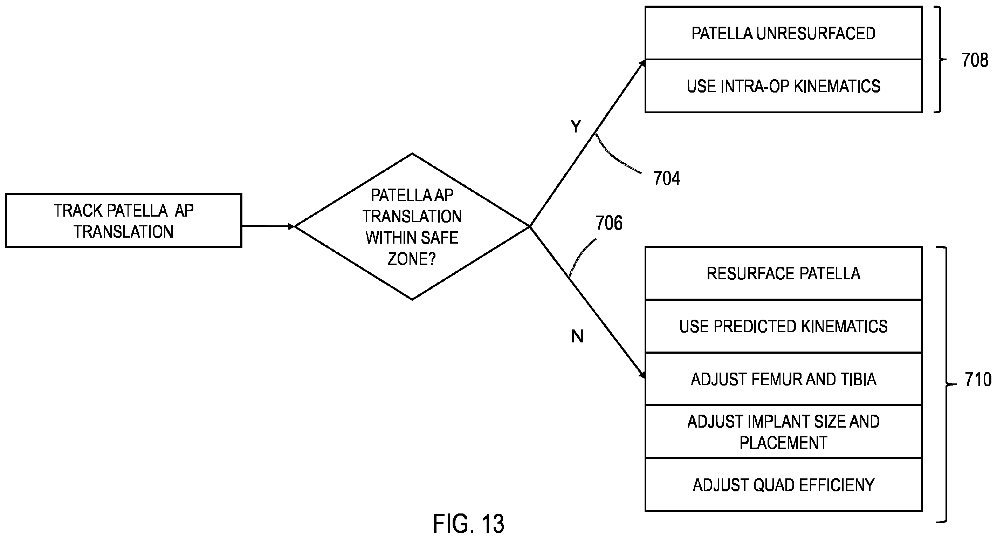

FIG. 13 presents a flowchart illustrating the steps to be taken when the tracked AP translation of the patella is either within the safe zone, as depicted by path 704, or outside the safe zone, as depicted by path 706. If the patella's AP translation falls within the safe zone, the surgeon may consider leaving the patella unresurfaced and using the actual live intra-operative kinematics as the intended surgical target, indicating optimal patellar motion without the need for further intervention (708).

Conversely, if the AP translation path is outside the safe limit, the surgeon can consider various corrective steps 710, as shown in FIG. 13. These steps include using predicted patellar kinematic paths instead of the natural kinematics measured, resurfacing the patella, and adjusting the AP position or implant type to achieve patellar balancing, and adjusting the sagittal position or rotation of the femur or tibia. Enhancing quadriceps efficiency can be necessary, which involves minimizing under-stuffing and avoiding patellofemoral pain by limiting over-stuffing to no more than 5 mm, for example. Furthermore, femur and tibia implant adjustments can be prioritized if the planned patella thickness with the implant is less than 20 mm or if the remaining patellar bone is less than 12 mm, ensuring a minimum thickness is maintained.

In cases where the native patella tracking is restored within safe zones with the unresurfaced patella and final femoral/tibial components, the surgeon may consider leaving the patella unresurfaced. However, if the native patella tracking is not restored within the safe zones, even with the unresurfaced patella and final femoral/tibial components, additional steps may be necessary. These can include restoring the native patella anatomy if the patella has already been resurfaced, considering patella resurfacing to adjust the AP position to restore native patella anatomy, performing recuts to adjust the sagittal position or rotation of the femur or tibia slope, or adjusting the tibial insert thickness, and enhancing quadricep efficiency.

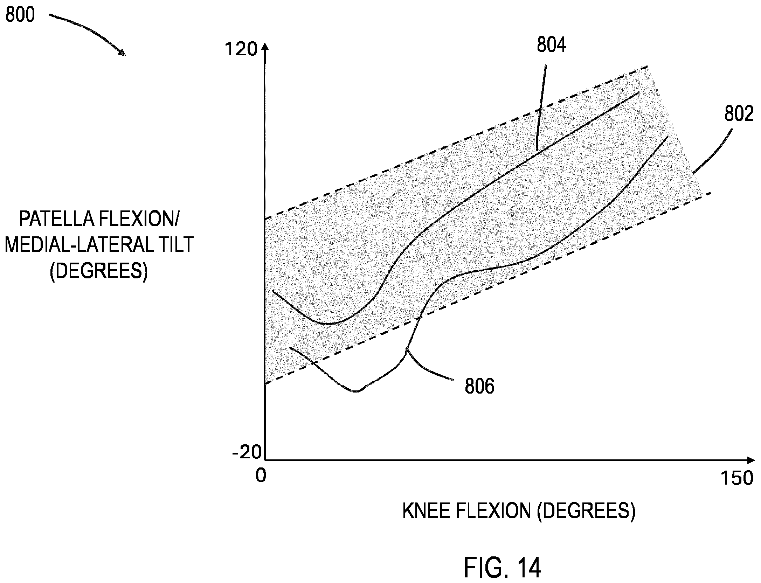

FIG. 14 shows a graph 800 depicting a medial-lateral tilt of the patella during knee flexion and extension, according to another embodiment of this disclosure. The graph tracks the movement of the patella and/or patellar implant as the knee flexes from 0 to 150 degrees, providing kinematic data on the medial-lateral tilt for the surgical system. The system monitors these movements and produces distinct paths, 804 and 806, as shown in FIG. 14. Path 804 indicates the medial-lateral tilt of the patella that remains within a designated safe zone 802 throughout the knee's range of motion. This suggests that the patella's movement along path 804 is optimal, ensuring proper kinematics and reducing the risk of complications such as patellar maltracking. In contrast, path 806 depicts the medial-lateral tilt of the patella deviating from the safe zone 802 at certain points, indicating suboptimal patellar movement. This deviation necessitates additional surgical intervention to realign the patella's motion to the ideal kinematic parameters. Such interventions may include adjusting the implant's position, size, or type, or further modifying the surgical plan to achieve the desired patellar kinematics.

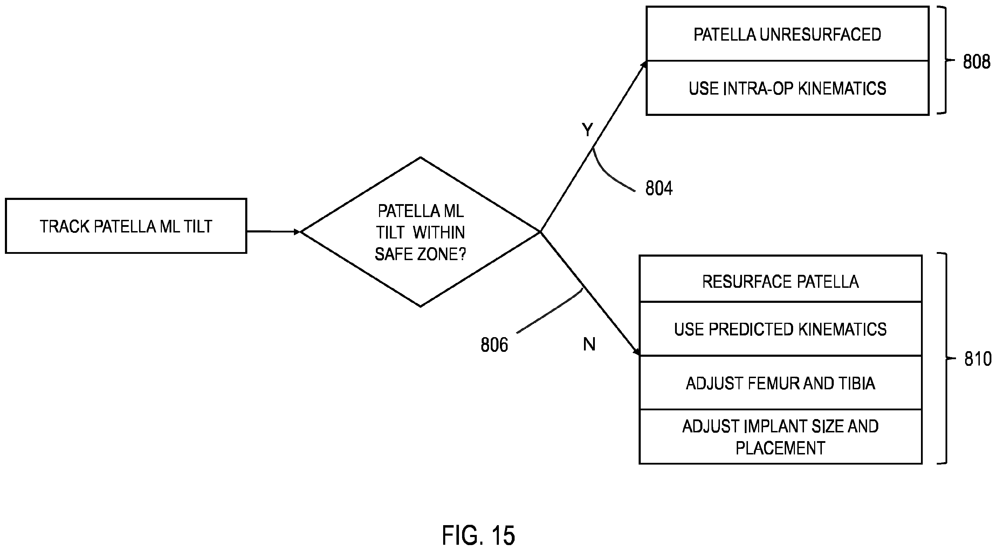

FIG. 15 is a flowchart illustrating the steps to be taken based on the measured ML tilt. If the ML tilt fall within the safe zones, the surgeon may use actual live intra-operative kinematics and consider leaving the patella unresurfaced (808).

If the patella is unhealthy or its ML tilt fall outside of the safe zones, the surgeon can take several corrective steps 810. These include using predicted AI/ML patellar kinematic paths, considering patella resurfacing and adjusting the patellar flexion or resection depth, or changing the patellar implant type to anatomical or dome. Additionally, the surgeon may consider adjusting the flexion of the femur or balancing the knee using posterior cruciate ligament (PCL) or femoral or tibial insert implant types based on anterior-posterior (AP) stability.

When native patella tracking is restored within safe zones with the unresurfaced patella and final femoral/tibial components, the surgeon may consider leaving the patella unresurfaced. However, if native patella tracking is not restored within safe zones with the unresurfaced patella and final femoral/tibial components, further steps are required. These can include patella resurfacing to adjust the patellar flexion or resection depth, changing the patellar implant type to anatomical or dome, adjusting the flexion of the femur, balancing the knee using PCL or femoral or tibial insert implant types based on AP stability, and using more AP stabilizing components if patellar flexion is low.

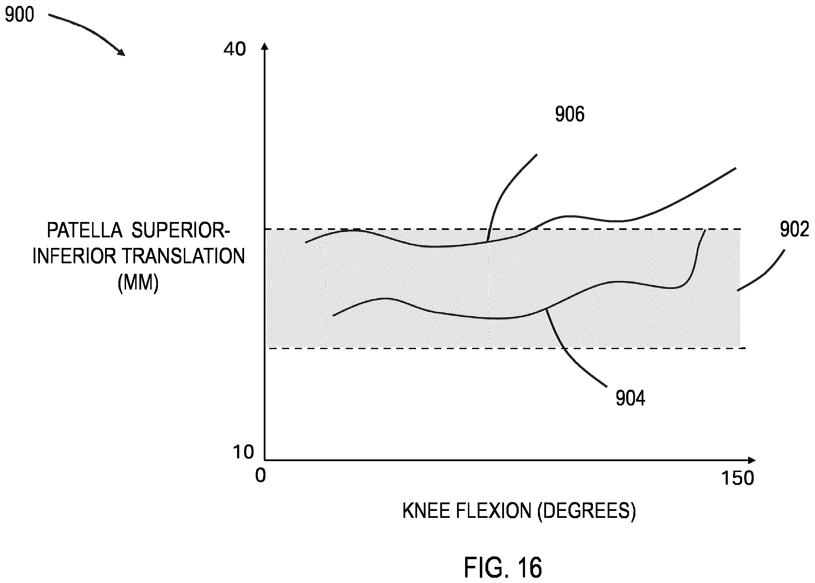

Graph 900 shown in FIG. 16, depicts a superior-inferior (SI) translation of the patella in relation to knee flexion and extension according to another embodiment of the present disclosure. As the knee flexes from 0 degrees to 150 degrees, the surgical system tracks the movement of the patella and/or patellar implant, providing kinematic data for the surgical system. The present disclosure's surgical system monitors the patella's trackers, generating distinct paths 904 and 906 depicting the patella's SI translation, as shown in FIG. 16. Path 904, which represents the superior-inferior translation of the patella, stays within a designated safe zone 902 throughout the range of knee flexion and extension. This indicates that the patella's movement along path 904 is within acceptable limits, ensuring proper patellar kinematics and reducing the risk of complications like patellar maltracking. In contrast, path 906 shows the superior-inferior translation of the patella deviating from the safe zone 902 in certain areas. Such deviations suggest that the patellar movement along path 706 is suboptimal, necessitating additional surgical intervention to align the patella's motion within ideal kinematic parameters. To achieve the desired patellar kinematics, this might involve adjusting the implant position, size, or type, or making further modifications to the surgical plan.

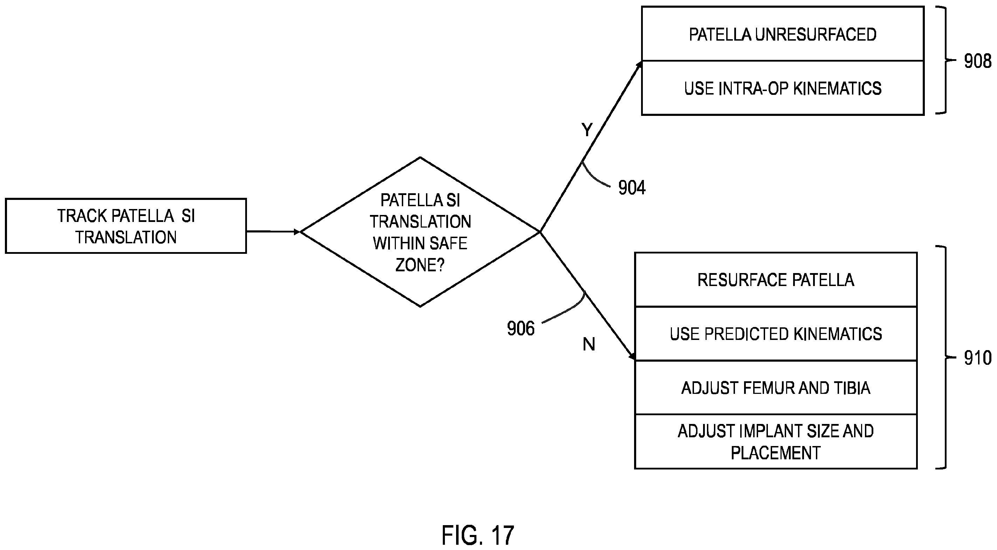

FIG. 17 is a flowchart illustrating the steps to be taken when the tracked SI translation of the patella is either within the safe zone, as depicted by path 904, or outside the safe zone, as depicted by path 906.

If the native patella's SI translation falls within the safe zone, the surgeon can use actual live patellar intra-operative kinematics and may consider leaving the patella unresurfaced, using these measured kinematics as the intended surgical target (908).

If the SI translation path is outside the safe zone, various corrective steps 910 can be considered. These steps can include using predicted patellar kinematic paths instead of the natural kinematics measured. The surgeon may also make decisions to correct patella alta or baja, consider patellar gaps in flexion influenced by patellar offset (AP translation) and distal femoral position, or opt for patella resurfacing. Adjustments might involve the superior-inferior (SI) position of the patella, selecting an appropriate implant type, or considering tibiofemoral (TF) knee balancing through femur distalization.

During femoral/tibial trialing, if native patella tracking is restored within the safe zones with an unresurfaced patella and final femoral/tibial components, the surgeon may consider leaving the patella unresurfaced. Conversely, if native patella tracking is not restored and remains outside the safe zones, steps to restore native patella anatomy, such as correcting patella alta/baja, adjusting the SI position of the patella, or resurfacing the patella may be necessary. Consideration can also be given to patellar gaps in flexion influenced by patellar offset and distal femoral position, as well as TF knee balancing.

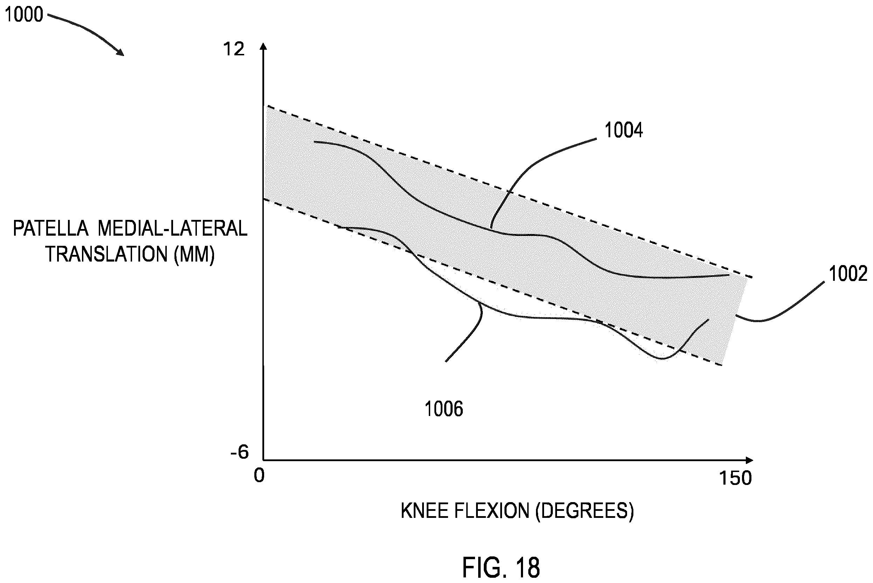

FIG. 18 is a graph 1000 showing a medial-lateral translation of the patella during knee flexion and extension, according to another embodiment of this disclosure. The surgical system tracks the movement of the patella and/or patellar implant as the knee flexes from 0 to 150 degrees, providing ML translation data for the surgical system. The system generates two distinct paths, 1004 and 1006, as depicted in FIG. 18.

Path 1004 indicates the medial-lateral translation of the patella that stays within the designated safe zone 1002 throughout the entire range of knee motion. This suggests that the patella's movement along path 1004 is optimal, ensuring proper kinematics and minimizing the risk of complications such as patellar maltracking. Conversely, path 1006 shows the medial-lateral translation of the patella deviating from the safe zone 1002 at certain points, signaling suboptimal patellar movement. Such deviations necessitate further surgical intervention to realign the patella's motion with the ideal kinematic parameters.

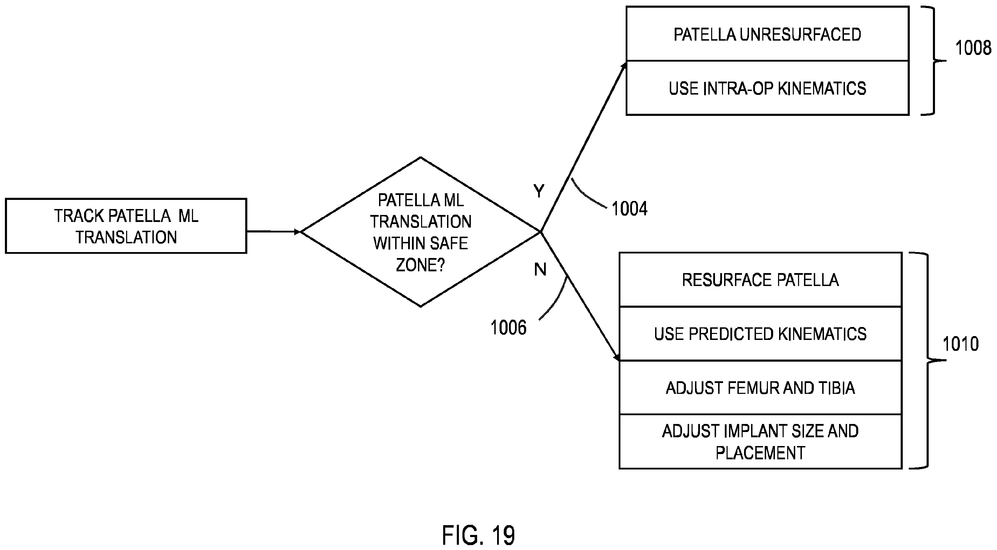

Interventions to correct this deviation may include adjusting the implant's position, size, or type, or further refining the surgical plan as show in a flowchart (FIG. 19). These adjustments ensure that the patella's motion aligns with the desired kinematic outcomes, ultimately enhancing the success of the knee arthroplasty and improving patient outcomes.

If the native patella's ML translation falls within the safe zone, the surgeon can use actual live patellar intra-operative kinematics and may consider leaving the patella unresurfaced, using these measured kinematics as the intended surgical target (1008).

If the ML translation path is outside the safe zone, various corrective steps 1010 can be considered. These steps can include using predicted patellar kinematic paths instead of the natural kinematics measured. The surgeon may also consider patella resurfacing by adjusting the medial-lateral (ML) position of the patella or selecting a different patellar implant type. Additionally, adjustments might involve correcting ML position or varus/valgus (V/V), internal/external (I/E) rotations of the femur, or considering tibiofemoral (TF) knee balancing, particularly if the lateral component is too tight, which can cause lateral maltracking.

During femoral/tibial trialing, if native patella tracking is restored within the safe zones with an unresurfaced patella and final femoral/tibial components, the surgeon may consider leaving the patella unresurfaced. Conversely, if native patella tracking is not restored and remains outside the safe zones, steps to restore native patella anatomy may be necessary. These steps include patella resurfacing, adjusting the ML position of the patella, or correcting V/V, I/E rotations of the femur. Consideration can also be given to patellar gaps in flexion influenced by patellar offset and distal femoral position, as well as TF knee balancing.

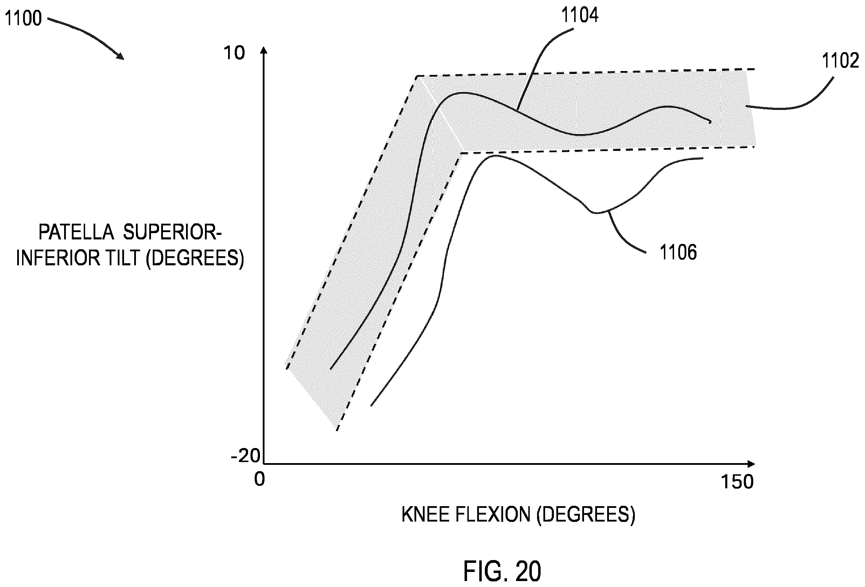

FIG. 20 shows a graph 1100 illustrating the superior-inferior tilt of the patella during knee flexion and extension, according to another embodiment of this disclosure. This graph tracks the movement of the patella and/or patellar implant as the knee flexes from 0 to 150 degrees, providing kinematic data for the surgical system. The system generates and monitors two distinct paths, 1104 and 1106, in this embodiment as depicted in FIG. 20.

Path 1104 represents the superior-inferior tilt of the patella that remains within the designated safe zone 1102 throughout the entire range of knee motion. This indicates that the patella's movement along path 1104 is optimal, ensuring proper kinematics and minimizing the risk of complications such as patellar maltracking. The consistent alignment within the safe zone suggests that the surgical adjustments have successfully maintained the natural motion of the patella.

Conversely, path 1106 shows the superior-inferior tilt of the patella deviating from the safe zone 1102 at certain points. This deviation indicates suboptimal patellar movement, which could lead to complications and necessitates further surgical intervention. Such deviations may arise from improper implant positioning, incorrect sizing, or inadequate adjustments during the procedure. To address these issues, the surgeon may need to take additional corrective measures. These interventions could include adjusting the position, size, or type of the implant to better align with the desired kinematic parameters. Further modifications to the surgical plan might also be necessary to ensure the patella's motion is optimized.

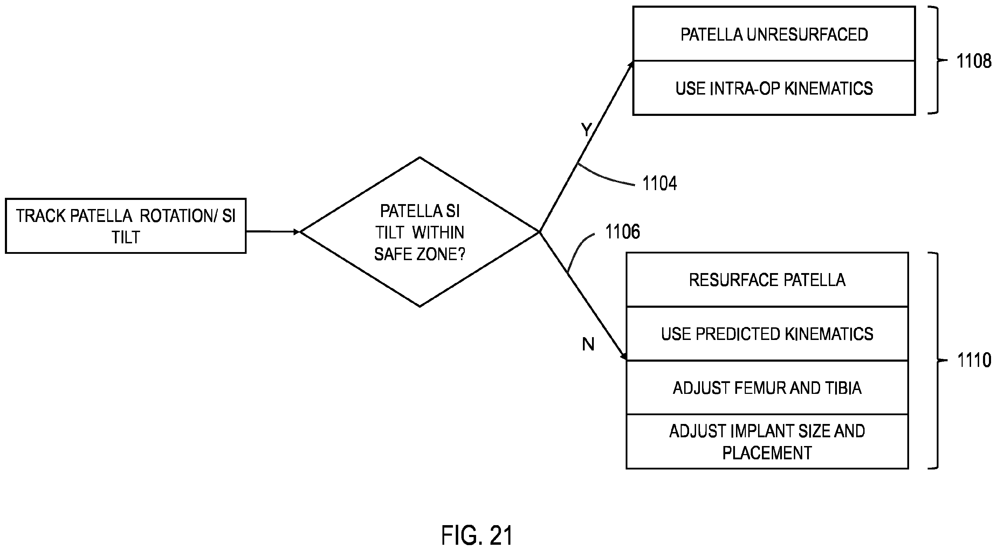

FIG. 21 is a flowchart illustrating the steps to be taken when the tracked superior-inferior (SI) translation of the patella is either within the safe zone, as depicted by path 1104, or outside the safe zone, as depicted by path 1106.

If the native patella's SI translation falls within the safe zone, the surgeon can use actual live patellar intra-operative kinematics and may consider leaving the patella unresurfaced, using these measured kinematics as the intended surgical target (1108).
if the SI translation path is outside the safe zone, various corrective steps 1110 can be considered. These steps can include using predicted AI/ML patellar kinematic paths instead of the natural kinematics measured. The surgeon might consider patella resurfacing by adjusting the medial-lateral (ML) position of the patella or selecting an appropriate patellar implant type. Consideration can also be given to adjusting the internal/external (I/E) rotation or resection depth of the patella, or choosing between an anatomical or dome implant type. In cases where passive assessment limitations impact surgical decisions, a measured resection approach to SI tilt can be considered.

During femoral/tibial trialing, if native patella tracking is restored within the safe zones with an unresurfaced patella and final femoral/tibial components, the surgeon may consider leaving the patella unresurfaced. Conversely, if native patella tracking is not restored and remains outside the safe zones, steps to restore native patella anatomy, such as adjusting patellar flexion or resection depth, resurfacing the patella, or selecting the appropriate implant type, may be necessary. Additionally, consideration should be given to the limitations on passive assessment and employing a "measured" resection approach to SI tilt.

By closely monitoring these kinematic paths and making precise adjustments, the surgical system aims to achieve the ideal patellar kinematics, thereby enhancing the overall success of the knee arthroplasty procedure and improving patient outcomes.

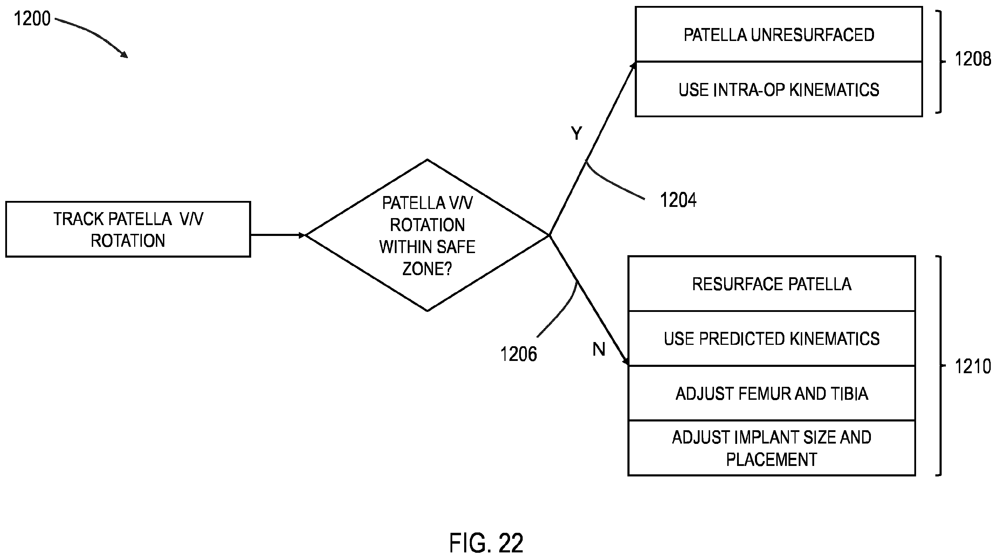

FIG. 22 is a flowchart illustrating the steps to be taken when tracked varus-valgus (V/V) rotation of the patella is either within a safe zone, as depicted by a path 1204, or outside the safe zone, as depicted by a path 1206.

If the native patella's V/V rotation falls within the safe zone, the surgeon can use actual live patellar intra-operative kinematics and may consider leaving the patella unresurfaced, using these measured kinematics as the intended surgical target (1208).

If the V/V translation path is outside the safe zone, various corrective steps 1210 can be considered. These steps include using predicted AI/ML patellar kinematic paths. The surgeon may also consider patella resurfacing or adjusting the varus/valgus (V/V) rotation, when considering anatomical patellar implant types.

During femoral/tibial trialing, if native patella tracking is restored within the safe zones with an unresurfaced patella and final femoral/tibial components, the surgeon may consider leaving the patella unresurfaced. Conversely, if native patella tracking is not restored and remains outside the safe zones, steps to restore native patella anatomy, such as patella resurfacing or adjusting the V/V (spin) rotation, may be necessary. These adjustments can be considered for anatomical patellar implant types.

Furthermore, although the invention disclosed herein has been described with reference to particular features, it is to be understood that these features are merely illustrative of the principles and applications of the present invention. It is therefore to be understood that numerous modifications, including changes in the sizes of the various features described herein, may be made to the illustrative embodiments and that other arrangements may be devised without departing from the scope of the present invention. In this regard, the present invention encompasses numerous additional features in addition to those specific features set forth in the paragraphs below. Moreover, the foregoing disclosure should be taken by way of illustration rather than by way of limitation as the present invention is defined in the examples of the numbered paragraphs, which describe features in accordance with various embodiments of the invention, set forth in the paragraphs below.

## Claims

1. A method for patella planning comprising:
tracking a location of a point adjacent an unresected patella in flexion and extension of a knee;
generating a native kinematic profile from the location of the point, the native kinematic profile representing a kinematic profile of the unresected patella, and
displaying and comparing the native kinematic profile with a target kinematic profile.

2. The method of claim 1, further including a step of using the native kinematic profile prior to bone resections if the native kinematic profile is within predetermined thresholds of the target kinematic profile.

3. The method of claim 1 or claim 2, wherein the step of generating the native kinematic profile includes displaying a line connecting locations of the tracked points on a display screen.

4. The method of any one of claims 1 to 3, wherein the native kinematic profile and the target kinematic profile represent patella anterior-posterior translation.

5. The method of any one of claims 1 to 4, wherein the native kinematic profile and the target kinematic profile represent patella medial-lateral translation.

6. The method of any one of claims 1 to 5, wherein the native kinematic profile and the target kinematic profile represent patella superior-inferior translation.

7. The method of any one of claims 1 to 6, wherein the native kinematic profile and the target kinematic profile represent patella medial-lateral tilt.

8. The method of any one of claims 1 to 7, wherein the native kinematic profile and the target kinematic profile represent patella superior-inferior tilt.

9. The method of any one of claims 1 to 8, wherein the native kinematic profile and the target kinematic profile represent patella varus-valgus rotation.

10. The method of any one of claims 1 to 9, wherein the target kinematic profile represents a kinematic profile of a healthy patella.

11. The method of any one of claims 1 to 10, wherein the step of tracking the location of the point adjacent the unresected patella includes tracking a plurality of points adjacent the unresected patella in flexion and extension of the knee.

12. The method of any one of claims 1 to 11, wherein the step of displaying and comparing the native kinematic profile with the target kinematic profile includes displaying and comparing the native kinematic profile with the target kinematic profile on a display screen.

13. The method of any one of claims 1 to 12, wherein the target kinematic profile is defined by predetermined thresholds.

14. The method of any one of claims 1 to 13, wherein the step of generating the native kinematic profile includes displaying multiple points representing locations of the tracked points on a display screen.
